(19) Europäisches Patentamt

European Patent Office

Office européen des brevets

(11) **EP 4 410 184 B1**

(12) **EUROPEAN PATENT SPECIFICATION**

(45) Date of publication and mention of the grant of the patent:
**11.06.2025 Bulletin 2025/24**

(21) Application number: **23162532.8**

(22) Date of filing: **17.03.2023**

(51) International Patent Classification (IPC):
*A61B 5/00* $^{(2006.01)}$ *A61B 5/318* $^{(2021.01)}$
*A61B 5/11* $^{(2006.01)}$

(52) Cooperative Patent Classification (CPC):
**A61B 5/4818; A61B 5/1117; A61B 5/318;**
**A61B 5/7267;** A61B 2560/0209

(54) **DETECTION METHOD OF EVENT, EVENT DETECTION SYSTEM AND INFERENCE SERVER**

EREIGNISDETEKTIONSVERFAHREN, EREIGNISDETEKTIONSSYSTEM UND INFERENZSERVER

PROCÉDÉ DE DÉTECTION D'ÉVÉNEMENT, SYSTÈME DE DÉTECTION D'ÉVÉNEMENT ET SERVEUR D'INFÉRENCE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **31.01.2023 TW 112103260**

(43) Date of publication of application:
**07.08.2024 Bulletin 2024/32**

(73) Proprietor: **Wistron Corporation**
**New Taipei City 22181 (TW)**

(72) Inventor: **HUANG, Yuan Fu**
**22181 New Taipei City (TW)**

(74) Representative: **Becker, Eberhard**
**Becker Kurig & Partner**
**Patentanwälte mbB**
**Bavariastraße 7**
**80336 München (DE)**

(56) References cited:
**CN-A- 110 876 621** **US-A1- 2011 251 985**
**US-A1- 2021 244 339** **US-A1- 2022 303 288**

**Description**

TECHNICAL FIELD

The invention relates to a machine learning technique, and in particular to a detection method of an event, an event detection system, and an inference server.

BACKGROUND

Sleep apnea refers to the symptoms of involuntary weakening or even cessation of breathing during sleep. The cessation of breathing is often unnoticed until the body is severely deprived of oxygen and wakes up due to discomfort. However, hypoxia causes damage to the body, and patients can even die suddenly due to cardiovascular disease after a long period of time. It's worth noting that the effects of sleep apnea go beyond daytime inattention and tiredness. Since the heart rate slows down and blood pressure drops during normal sleep, normal sleep gives the cardiovascular system a chance to rest and recover. However, sleep in apnea patients can be interrupted several times a night, depriving them of cardiovascular rest. In addition, repeated apnea can cause blood oxygen levels to drop, cause a certain amount of stress on the body and lead to a systemic inflammatory response, and can also increase the risk of cardiovascular disease. People with sleep apnea are often unaware of symptoms. Symptoms can only be discovered when the patient goes to the hospital for detection and diagnosis with special equipment. Researchers estimate that about 80 percent of people with moderate to severe sleep apnea are undiagnosed. In addition, sleep apnea is involved in a wide range of levels, such as traffic accidents, industrial safety accidents, cardiovascular diseases, metabolic diseases, etc.

Moreover, events such as falls, car accidents, etc., are accidents that humans can encounter. If these accidents are detected early, the damage can be minimized.

US2022/303288A1, CN110876621A and US2021/244339A1 define systems for event detection and data compression.

SUMMARY OF THE INVENTION

Accordingly, the embodiments of the disclosure provide a detection method of an event, an event detection system, and an inference server that can detect sleep quality and an unexpected event.

An event detection system of an embodiment includes (but not limited to) one or more terminal devices and an inference server. The terminal device generates first compressed data. The first compressed data is related to a sensing result of a physiological state or a motion state. The inference server decodes the first compressed data into reconstructed data via a decoder in an anomaly detection model, encodes the reconstructed data into second compressed data via an encoder, and determines an event of the physiological state or the motion state by an error between the first compressed data and the second compressed data. The anomaly detection model includes the encoder and the decoder.

A detection method of an event of an embodiment includes (but not limited to) the following steps. First compressed data is received. The first compressed data is related to a sensing result of a physiological state or a motion state. The first compressed data is decoded into reconstructed data via a decoder in an anomaly detection model. The anomaly detection model includes the decoder and an encoder. The reconstructed data is encoded into second compressed data via the encoder. An event of the physiological state or the motion state is determined by an error between the first compressed data and the second compressed data.

An inference server of an embodiment includes (but not limited to) a communication transceiver, a memory, and a processor. The communication transceiver transmits or receives data. The memory stores a program code. The processor loads the program code to execute: receiving first compressed data via the communication transceiver, decoding first compressed data into reconstructed data via a decoder in an anomaly detection model, encoding the reconstructed data into second compressed data via an encoder in the anomaly detection model, and determining an event of a physiological state or a motion state by an error between the first compressed data and the second compressed data. The first compressed data is related to a sensing result of the physiological state or the motion state. The anomaly detection model is based on an autoencoder, and the anomaly detection model includes the decoder and the encoder.

Based on the above, according to the detection method and the event detection system and the inference server of the embodiments , the terminal device only transmits compressed data, and the inference server determines events based on the compressed data from the terminal device and the compressed data converted by the decoder and the compressor of the anomaly detection model. In this way, bandwidth can be effectively utilized, power can be saved, and event detection with data privacy can be provided.

In order to make the aforementioned features and advantages of the disclosure more comprehensible, embodiments accompanied with figures are described in detail below. The invention is defined in the appended claims.

BRIEF DESCRIPTION OF THE DRAWINGS

FIG. 1 is a block diagram of elements of a detection system according to an embodiment .

FIG. 2 is a flowchart of a detection method of an event according to an embodiment

FIG. 3 is a timing diagram illustrating an example of a heartbeat.

FIG. 4 is a schematic diagram of an autoencoder according to an embodiment

FIG. 5 is a schematic diagram of an anomaly detection model according to an embodiment

FIG. 6 is a schematic diagram of an anomaly detection model according to another embodiment

FIG. 7 is a flowchart of event determination according to an embodiment

DESCRIPTION OF THE EMBODIMENTS

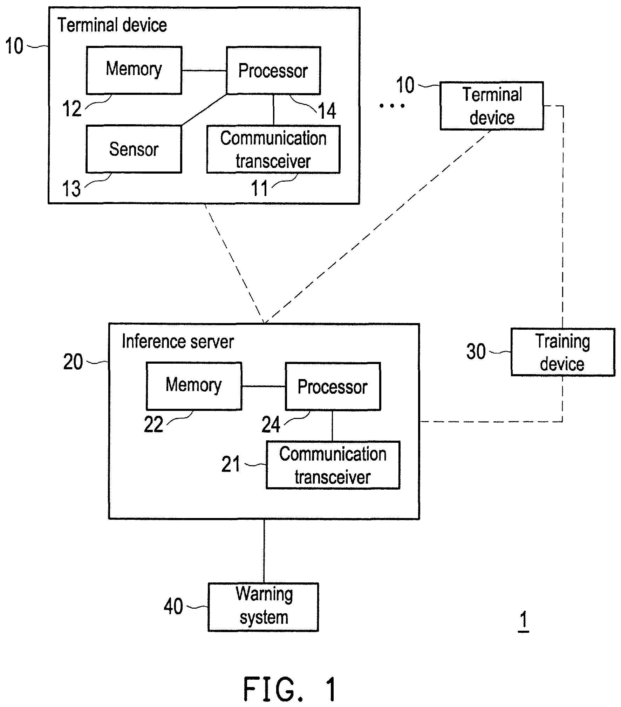

FIG. 1 is a block diagram of elements of an event detection system 1 according to an embodiment. Referring to FIG. 1, the event detection system 1 includes (but not limited to) one or more terminal devices 10, an inference server 20, a training device 30, and a warning system 40.

The terminal device 10 can be an IoT device, a wearable device, a medical testing instrument, a smart phone, a tablet computer, or a sensing device.

The terminal device 10 includes (but not limited to) a communication transceiver 11, a memory 12, a sensor 13, and a processor 14.

The communication transceiver 11 can be a communication transceiver circuit supporting low power wide area network (LPWAN) techniques (LPWAN communication techniques such as long distance (LoRa) techniques, Narrow Band Internet of Things (NB-IoT), Sigfox, LTE Advanced for Machine Type Communications (LTE-MTC)), and it can also be a communication transceiver circuit or a transmission interface card supporting Wi-Fi, Bluetooth, mobile communication, USB, or Ethernet. In an embodiment, the communication transceiver 11 transmits or receives data with an external device (e.g., the inference server 20 or the training device 30).

The memory 12 can be any form of a fixed or movable random-access memory (RAM), read-only memory (ROM), flash memory, traditional hard disk drive (HDD), solid-state drive (SSD), or similar devices. In an embodiment, the memory 12 stores a program code, a software module, a configuration, data, or a file (for example, compress data, a sensing result, or a feature), and is described in detail in subsequent embodiments.

The sensor 13 can be a heartbeat sensor, a physiological sensor, an image sensor, a motion sensor, or other types of sensors. In an embodiment, the sensor 13 senses a physiological state (e.g., heartbeat, respiration rate, or blood oxygen level) or a motion state (e.g., inertial attitude, acceleration, or moving direction) to obtain a sensing result. In an embodiment, the sensing result is time-dependent data. That is, data recorded with time sequence, continuous time, or multiple points in time. For example, the sensing result is an electrocardiography (ECG), respiratory airflow in a polysomnography (PSG) of a night's sleep, chest movement, abdominal muscle behavior, or an electroencephalogram.

The processor 14 is coupled to the communication transceiver 11, the memory 12, and the sensor 13. The processor 14 can be a central processing unit (CPU), a graphics processing unit (GPU), or other programmable general-purpose or special-purpose microprocessors, digital signal processors (DSPs), programmable controllers, field-programmable gate arrays (FPGAs), application-specific integrated circuits (ASICs), neural network accelerators, or other similar devices or a combination of the above devices. In an embodiment, the processor 14 performs all or part of the operations of the terminal device 10, and can load and execute each of the program codes, software modules, files, and data stored in the memory 12.

The inference server 20 can be a cloud/edge computing server, a workstation, or a computer.

The inference server 20 includes (but not limited to) a communication transceiver 21, a memory 22, and a processor 24.

The implementation and functions of the communication transceiver 21 are as provided in the description of the communication transceiver 11 and are not repeated herein. In an embodiment, the communication transceiver 21 transmits or receives data with an external device (e.g., the warning system 40 or the training device 30).

The implementation and functions of the memory 22 are as provided in the description of the memory 12 and are not repeated herein. In an embodiment, the memory 22 stores data such as compressed data, reconstructed data, errors, or determination results, which is described in detail in subsequent embodiments.

The processor 24 is coupled to the communication transceiver 21 and the memory 22. The implementation and functions of the processor 24 are as provided in the description of the processor 14 and is not repeated herein. In an embodiment, the processor 24 performs all or part of the operations of the inference server 20, and can load and execute each of the program codes, software modules, files, and data stored in the memory 22. In some embodiments, some operations in a method of an embodiment can be implemented by different or the same processor 24.

The training device 30 is communicatively connected to the terminal device 10 and the inference server 20. The training device 30 can be a smart phone, a tablet, a computer, a server, or a workstation. In an embodiment, the training device 30 trains a model based on a machine learning algorithm (e.g., autoencoder, neural network, decision tree, or random forest). The machine learning algorithm can analyze training samples to obtain patterns therefrom, so as to predict unknown data via the patterns. For example, the machine learning model establishes the association between the nodes in the hidden layer between feature data (i.e., the input of the model) and a respiratory event (i.e., the output of the model) by the labeled samples (e.g., feature data of known hypopnea/apnea events, or feature data of known normal breathing events). The machine learning model is a model constructed after learning, and can accordingly infer data to be evaluated (for example, feature data or compressed data).

The warning system 40 is communicatively connected to the inference server 20. The warning system 40 can be a display, a speaker, or a communication transceiver. In an embodiment, the warning system 40 sends out a warning message.

In some embodiments, the functions of the training device 30 and/or the warning system 40 can also be implemented via the inference server 20, or these devices are integrated into a single device.

Hereinafter, the method described in an embodiment is described in conjunction with various devices and elements in the event detection system 1. Each of the processes of the present method can be adjusted according to embodiment conditions and is not limited thereto.

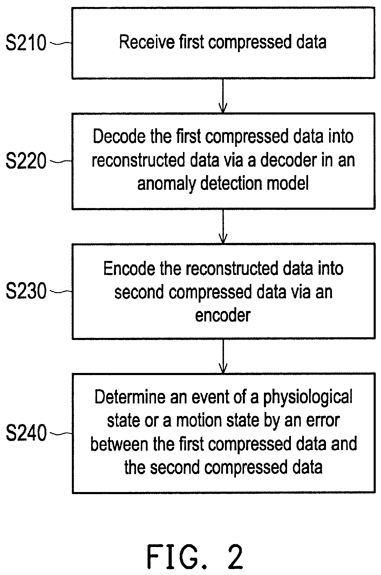

FIG. 2 is a flowchart of a detection method of an event according to an embodiment. Referring to FIG. 2, the processor 24 of the inference server 20 receives first compressed data via the communication transceiver 21 (step S210). Specifically, the processor 14 of the terminal device 10 generates the first compressed data by the sensing result (i.e., raw data) of the sensor 13 for a physiological device or a motion device. In other words, the first compressed data is related to the sensing result of the physiological state or the motion state.

In an embodiment, the physiological state is a heartbeat. For example, the sensing result of the heartbeat is the R-R interval of a heartbeat waveform in a time sequence. It is worth noting that each person's heart rate is different, and under normal circumstances, the normal adult heart rate is 60 to 100 beats per minute. A normal person's heart rate is affected by many factors. The heart beats faster when exercising, slows down when resting or sleeping, and slows down some more when exhaling. In addition, conditions such as fever, nervousness, stress, and pain can also affect heart rate. Heart rate is affected by breathing rate. Under normal circumstances, the heart rate of women is faster than that of men. A normal adult breathes about 16 to 20 times per minute. The ratio of the number of breaths to heart rate is 1:4, that is, for every breath, the heart beats four times. Therefore, heartbeat can be used as an indicator of a person's mood, exercise, and breathing conditions. According to the literature (https://www.ncbi.nlm.nih.gov/pmc/articles/PMC8590683/pdf/11325_2020_Article_22 49.pdf), the human respiratory condition can be detected by observing heart rate variability. Heart rate variability is as the acceleration and deceleration of the heart rhythm. Via such variation of the heart rhythm, it is also possible to understand the health condition of an individual. The terminal device 10 can collect the R-R interval of the heartbeat via ECG.

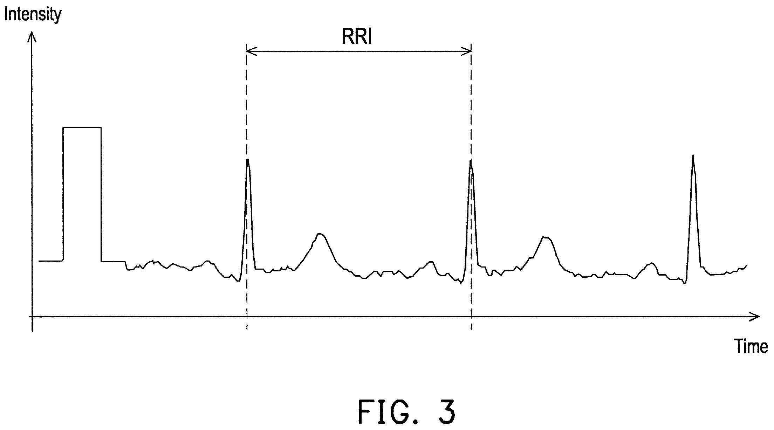

For example, FIG. 3 is a timing diagram illustrating an example of a heartbeat. Please refer to FIG. 3, generally speaking, a series of wave bands in the ECG include a P wave, a QRS complex, and a T wave. The QRS complex consists of a series of 3 deviations. The negative wave of the first deviation in the complex is called Q wave, the first positive deviation in the complex is called R wave, and the negative deviation after R wave is called S wave. The distance between two consecutive R waves can be called R-R interval RRI.

In an embodiment, the processor 14 of the terminal device 10 can encode the sensing result of the heartbeat into the first compressed data via an encoder. The anomaly detection model includes the decoder and the encoder. The abnormal detecting model is based on an autoencoder architecture, a machine learning architecture composed of the encoder and the decoder, or other neural network architectures reconstructing a sample or other specified samples.

In an embodiment, the training device 30 trains the anomaly detection model by the plurality of samples labeled as events. For example, the anomaly detection model for heartbeats can determine a sleep apnea event, wherein the waveform of the heartbeat can have significant rapid fluctuations when a sleep apnea event occurs. Medical testing equipment can mark R-R interval segments having a sleep apnea event. Assuming that

$$R^{\{i\}} = [R_1^{\{i\}}, \dots, R_t^{\{i\}}, \dots]^T$$ (i is one of 1 to I) is the training sample of the R-R interval in the time sequence, then $R_t^{\{i\}}$ is the t-th R-R interval value of the i-th patient having a sleep apnea symptom (that is, a sleep apnea event occurs) at the moment, and I represents the number of all apnea patients in the training sample. In an embodiment, in order to remove the variation of individual data, $R^{\{i\}}$ can be standardized to $\tilde{R}^{\{i\}}$, so that these training samples have the characteristics of the average value of 0 and the variation of 1:

$$\tilde{R}^{\{i\}} = [\tilde{R}_1^{\{i\}}, \dots, \tilde{R}_t^{\{i\}}, \dots]^T .$$

For the anomaly detection model of the autoencoder, the training device 30 can divide the time sequence data of the R-R interval labeled as the sleep apnea event into a plurality of RRI time sequence segments having the same samples. For example, if

$$\tilde{R}^{\{i\}} = [\tilde{X}_0^{\{i\}}, \dots, \tilde{X}_k^{\{i\}}, \dots]^T , \text{ and the number}$$

of samples is N, then $X_k^{\{i\}} = \left[ \tilde{R}_{(k-1)N}^{\{i\}}, \cdots, \tilde{R}_{KN}^{\{i\}} \right]^T$, and

$$\sum_{k=0}^{k=M} \#(\tilde{X}_k^{\{i\}}) \leq \#(\tilde{R}^{\{i\}})$$

. That is, an RRI sample with a sample number of $\#(\tilde{R}^{\{i\}})$ is cut into M segments, and each segment has N RRI samples. K represents the K-th segment RRI sample after segmentation, and the symbol # is the number of RRI samples.

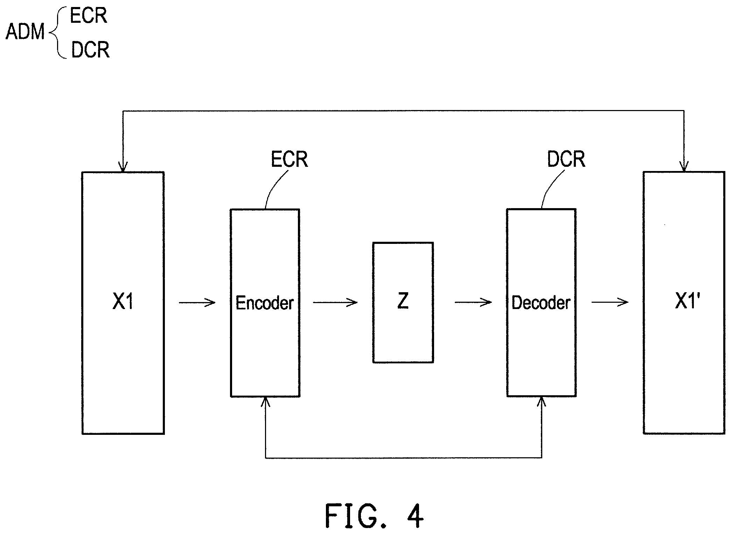

FIG. 4 is a schematic diagram of an autoencoder according to an embodiment. Please refer to FIG. 4, $X_k^{\{i\}}$ is input data X1 in an anomaly detection model ADM. Reconstructed data X1' is data compressed by an encoder ECR of the anomaly detection model ADM and decompressed by a decoder DCR. The number of samples of the reconstructed data X1' is the same as that of the input data X1. That is, if the number of samples of the input data X1 is N, the reconstructed data X1' also has N samples.

In the training of the anomaly detection model, the anomaly detection model ADM can be split into two neural networks, the encoder ECR and the decoder DCR. The input data is input to the encoder ECR, and is compressed into relatively low-dimensional compressed data Z1 (for example, a one-dimensional vector) by the encoder ECR. The compressed data Z1 is input to the decoder DCR, and the reconstructed data X1' having the same size as the input data X1 is restored by the decoder DCR. In order to make the reconstructed data X1' similar to or the same as the input data X1, mean-square error (MSE), mean absolute error (MAE), cross entropy, or focal loss can be used as the loss function. In addition, the training device 30 can update the weights in the neural network via backpropagation to minimize the loss function, and then train the abnormality detection model ADM.

The training device 30 can transmit the encoder ECR in the anomaly detection model ADM to the terminal device 10, and transmit the encoder ECR and decoder DCR in the anomaly detection model ADM to the inference server 20.

In another embodiment, the motion state is an inertial attitude. For example, motion index (e.g., velocity, angular velocity, or acceleration), orientation, or displacement on three or six axes. The processor 14 of the terminal device 10 can encode the sensing result of the inertial attitude into the first compressed data via an encoder. That is, the terminal device 10 can encode the sensing result into the first compressed data via the trained encoder ECR. The anomaly detection model for inertial attitude can determine a fall event or a car accident event, wherein when a fall event or a car accident event occurs, the intensity of the acceleration and the orientation of the attitude can be changed rapidly.

It should be noted that the types of events can still be changed according to the actual needs of the user.

Then, the processor 14 of the terminal device 10 can transmit the first compressed data to the inference server 20 via the communication transceiver 11.

In an embodiment, the processor 24 of the inference server 20 can receive the first compressed data from one or more terminal devices 10 via a low power wide area network (LPWAN) via the communication transceiver 21. LPWAN has the characteristics of long-distance communication and power saving, and LPWAN can solve the transmission issue of the Internet of Things. It is worth noting that, compared with mobile network standards that pursue larger bandwidth, higher speed, or lower latency but consume more power, there is a wider deployment range for machine-to-machine (M2M) communication, and the need for frequent battery replacement is avoided, and LPWAN techniques provide the characteristics of less data volume, long-distance transmission, and power saving, and therefore are suitable for the application field of Internet of Things. For example, for environmental monitoring or asset tracking, LPWAN techniques with a transmission distance of up to 20 kilometers can significantly reduce deployment costs, and only a few stations are needed to cover a large area. LPWAN techniques are, for example, LoRa, Sigfox, and NB-IoT. However, LPWAN emphasizes coverage and power saving, but has relatively limited bandwidth. For example, LoRa is only 100 bps, Sigfox is 300 bps to 50 kbps, and NB-IOT is 50 kbps. Under the limitation of limited bandwidth, it should be difficult to load a large number of terminal devices 10 for simultaneous transmission, and it is even more difficult to meet instant communication requirements such as health and industrial safety monitoring. However, in the embodiments the sensing result can be compressed, thus not only reducing the amount of data to effectively utilize bandwidth, but can also achieve the effect of data privacy.

In other embodiments, the network between the terminal device 10 and the inference server 20 can also be other communication techniques. For example, Wi-Fi or Bluetooth.

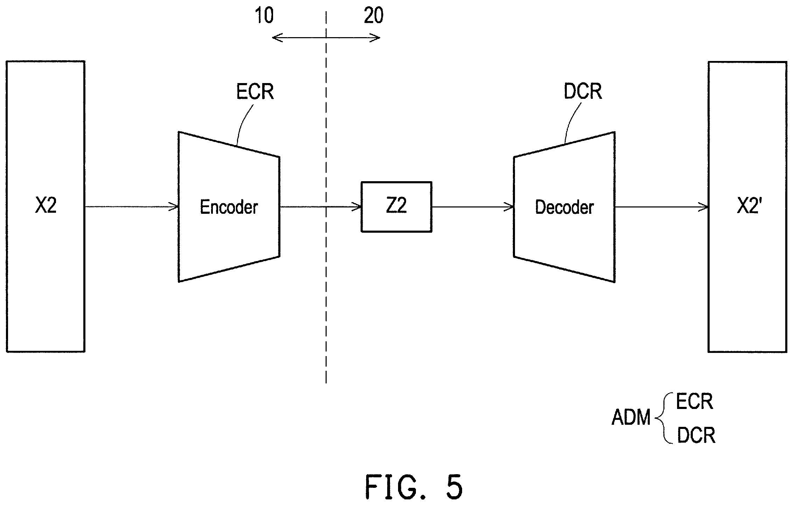

Referring to FIG. 2, the processor 24 of the inference server 20 decodes the first compressed data into reconstructed data via the decoder in the anomaly detection model (step S220). Specifically, FIG. 5 is a schematic diagram of an anomaly detection model ADM according to an embodiment. Please refer to FIG. 5, which is an architecture diagram of an autoencoder. One of the objects of the autoencoder is to find the functions $g_\phi$ and $f_\theta$ such that X2'= $f_\theta$ ($g_\phi$ (X2)), and X2≈X2'. $g_\phi$ is the mathematical function of the encoder ECR, and $f_\theta$ is the mathematical function of the decoder DCR. Compressed data Z2 (hereinafter referred to as the first compressed data) is the data obtained by compressing the input data X2 (i.e., the above sensing result) via the function $g_\phi$. In an embodiment the compression operation corresponding to the function $g_\phi$ can be im-

plemented via the terminal device 10, so that the first compressed data can be transmitted via the network.

Taking heartbeat time sequence data as an example, the input data X2 is a sequence of heartbeat time data (the number of samples can exceed a certain number (for example, 70, 100, or 200) or the sensing time can exceed a certain time (for example, 5 minutes, 30 minutes, or 1 hour)), and the first compressed data Z2 is the data after the input data X2 is compressed via the encoder ECR. The reconstruction data X2' is data decoded by the decoder DCR. To detect abnormal conditions (for example, the above events), the models of the functions $g_{\emptyset}$ and $f_{\theta}$ are trained using the heartbeat time sequence data of sleep apnea as described above. After training, the models of the encoder ECR and the decoder DCR can be used to detect whether there is a sleep apnea event within a period of time. If the input data X2 of the heartbeat time sequence is sequentially reconstructed by the encoder ECR and the decoder DCR in the anomaly detection model ADM, and the reconstructed data X2' is equal to or approximate to the input data X2, it is considered normal (i.e., a sleep apnea event occurs). Otherwise, it is considered abnormal (i.e., a sleep apnea event does not occur or a normal event occurs).

In an embodiment, the processor 24 can determine whether the error between the input data X2 and the reconstructed data X2' (for example, |X2-X2'|) is less than a first threshold. The first threshold can be obtained by data occupying a specific quantile (e.g., 97.5, 95, or 90) in the data distribution of all training samples in which a sleep apnea event occurs. In response to the error between the input data X2 and the reconstructed data X2' being less than the first threshold, the processor 24 can determine that an event (such as a sleep apnea event) occurs. In response to that the error between the input data X2 and the reconstructed data X2' is not less than the first threshold, the processor 24 can determine that an event does not occur (for example, a sleep apnea event does not occur or a normal event occurs).

In an embodiment the decoding operation corresponding to the function $f_{\theta}$ can be implemented via the inference server 20. It should be noted that, in the architecture of FIG. 5, the terminal device 10 still needs to transmit the input data X2 for the inference server 20 to compare the reconstructed data X2'. In other words, the terminal device 10 needs to transmit both the input data X2 and the compressed data Z2, thus affecting bandwidth usage and cannot achieve the effect of data protection.

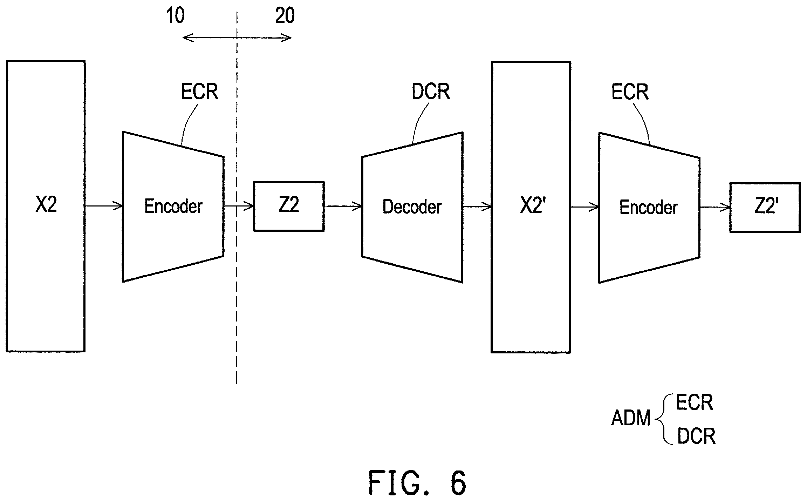

Referring to FIG. 2, the processor 24 of the inference server 20 encodes the reconstructed data into second compressed data via the encoder of the anomaly detection model (step S230). Specifically, FIG. 6 is a schematic diagram of the anomaly detection model ADM according to another embodiment. Please refer to FIG. 6, compared with the architecture in FIG. 5, the inference server 20 also implements the compression operation corresponding to the function $g_{\emptyset}$. That is, the

encoder ECR encodes the reconstructed data X2' into compressed data Z2' (hereinafter referred to as second compressed data). If the second compressed data Z2' of the input data X2 sequentially processed by the encoder ECR, the decoder DCR, and the encoder ECR in the anomaly detection model ADM is equal to or approximate to the first compressed data Z2, it is considered normal (for example, a sleep apnea event occurs, a fall event occurs, or a car accident event occurs). Otherwise, it is considered abnormal (e.g., a sleep apnea event does not occur or a normal event occurs). It can be seen from this that the terminal device 10 only needs to transmit the first compressed data Z2 to the inference server 20.

Referring to FIG. 2, the processor 24 determines the event of the physiological state or the motion state by the error between the first compressed data and the second compressed data (step S240). In an embodiment, the event determined by the processor 24 by the error between the first compressed data and the second compressed data refers to a predicted event output by the inference server 20. That is, the prediction result of the anomaly detection model is an event of the physiological state or the motion state.

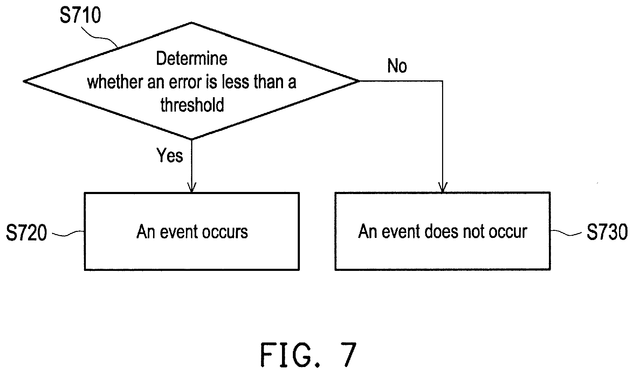

Specifically, FIG. 7 is a flowchart of event determination according to an embodiment of the invention. Referring to FIG. 7, the processor 24 can determine whether the error between the first compressed data Z2 and the second compressed data Z2' (for example, |Z2-Z2'|) is less than a second threshold (step S710). The second threshold can be obtained by data occupying a specific quantile (e.g., 97.5, 95, or 90) in the data distribution of all training samples in which a sleep apnea event occurs. In response to the error between the first compressed data Z2 and the second compressed data Z2' being less than the second threshold, the processor 24 can determine that an event occurs (such as a sleep apnea event) (step S720). In response to the error between the first compressed data Z2 and the second compressed data Z2' being not less than the second threshold, the processor 24 can determine that an event does not occur (such as a sleep apnea event does not occur or a normal event occurs) (step S730).

Similarly, for the determination of a fall, a car accident, or other events, it is also possible to determine whether the event occurs by the error between the first compressed data Z2 and the second compressed data Z2', and details are not repeated herein.

In other embodiments, if the training phase utilizes training samples labeled as normal events (e.g., a sleep apnea event does not occur, a normal breathing event does not occur, a fall event does not occur, or a car accident event does not occur), then it can also be that an abnormal event occurs when the error between the first compressed data and the second compressed data is not less than the corresponding threshold (for example, a sleep apnea event occurs, a fall event occurs, or a car accident occurs). When the error between the first compressed data and the second compressed data is less than the corresponding threshold, there is an abnormal event (for example, a sleep apnea event does not occur, a normal breathing event occurs, a fall event does not occur, or a car accident event does not occur).

For example, normal events refer to time sequence segments marked as free of anomalies. The features of these time sequence segments are usually distributed within a certain range. It is assumed that there is a time sequence data with a Gaussian distribution. If the data to be evaluated is detected to be located within 95% of the standard deviation of the mean value of the Gaussian distribution, the data to be evaluated is usually determined to come from the Gaussian distribution (regarded as normal). On the contrary, if located outside 95% of the standard deviation, the data to be evaluated is identified as abnormal. From the concept of distance, normal data is closer to the data in the data distribution of the normal data. Conversely, abnormal data is compared to data in a data distribution farther than the normal data. If the model is trained on the normal data, this model means that the prediction thereof of the normal data is relatively more accurate. That is, when the normal data goes through the anomaly detection model, the compressed and restored value (that is, the reconstructed data) is closer to the original input value. If the abnormal data is brought into this abnormal detection model, it is difficult to restore the abnormal data via compression. Therefore, the distance between the abnormal data and the restored data is farther. The first threshold and second threshold are based on this concept.

It should be noted that, for different terminal devices, the inference server 20 can deploy corresponding anomaly detection models respectively. In other words, for the first compressed data from one terminal device 10, a set of decoders and encoders is deployed; and for the first compressed data from another terminal device 10, another set of decoders and encoders is deployed. However, the deployment of the anomaly detection models is not limited to one-to-one relationships. For example, the inference server 20 runs fewer anomaly detection models concurrently, but with prioritization.

In an embodiment, the inference server 20 can request the warning system 40 to send a warning message for events such as falls, sleep apnea, or car accidents. For example, sending a push notification, making a phone call to the relevant authorities, or sounding a warning. In addition, the inference server 20 can also transmit the determination result to the warning system 40 periodically or based on a trigger event. The warning system 40 can report the overall health status.

Based on the above, in the detection method of event, the event detection system, and the inference server of the embodiments the terminal device transmits the first compressed data to the inference server. In addition to the decoder, the inference server further generates the second compressed data via the decoder. Ultimately, it is only necessary to compare the first compressed data and the second compressed data to determine whether an event occurs. In this way, energy and electricity can be lowered, data transmission is reduced, and bandwidth is lowered, so as to be suitable for IoT networks and implement long-term event monitoring. In addition, the transmission of compressed data across networks can provide data privacy.

## Claims

1. An event detection system (1), wherein the event detection system (1) comprises:

    a terminal device (10) encoding a sensing result of a physiological state or a motion state into first compressed data (Z2) via an encoder (ECR); and
    an inference server (20):

        receiving the first compressed data (Z2) via a network;
        decoding the first compressed data (Z2) into reconstructed data (X2') via a decoder (DCR) in an anomaly detection model (ADM); wherein the anomaly detection model (ADM) comprises the decoder (DCR) and the encoder (ECR);
        encoding the reconstructed data (X2') into second compressed data (Z2') via the encoder (ECR); and
        determining an event of the physiological state or the motion state by an error between the first compressed data (Z2) and the second compressed data (Z2').

2. The event detection system (1) of claim 1, **characterized in that** the physiological state is a heartbeat, and the terminal device (10) further:

    encodes a sensing result of the heartbeat into the first compressed data (Z2) via the encoder (ECR); wherein the event is a sleep apnea event, wherein the sensing result of the heartbeat is an R-R interval (RRI) of a heartbeat waveform in a time sequence; or
    the motion state is an inertial attitude, and the terminal device (10) further:
    encodes a sensing result of the inertial attitude into the first compressed data (Z2) via the encoder (ECR); wherein the event is a fall event.

3. The event detection system (1) of any one of claims 1-2, **characterized in that** the inference server (20) further:

    determines whether the error is less than a threshold; wherein the threshold is obtained by a plurality of samples labeled as the event; the event occurs in response to the error being less than the threshold; and the event does not occur in response to the error not being less than the threshold.

4. The event detection system (1) of any one of claims 1-3, **characterized in that** the inference server (20) further: receives the first compressed data (Z2) from the terminal device (10) via a low power wide area network, named LPWAN.

5. The event detection system (1) of any one of claims 1-4, **characterized in that** the event detection system (1) further comprises: a training device (30) training the anomaly detection model (ADM) by a plurality of samples labeled as the event; wherein the anomaly detection model (ADM) is trained based on an autoencoder.

6. A detection method of an event, wherein the detection method comprises:

   encoding a sensing result of a physiological state or a motion state into first compressed data (Z2) via an encoder (ECR);
   receiving the first compressed data (Z2) via a network;
   decoding the first compressed data (Z2) into reconstructed data (X2') via a decoder (DCR) in an anomaly detection model (ADM); wherein the anomaly detection model (ADM) is based on an autoencoder, and the anomaly detection model (ADM) comprises the decoder (DCR) and the encoder (ECR);
   encoding the reconstructed data (X2') into second compressed data (Z2') via the encoder (ECR); and
   determining an event of the physiological state or the motion state by an error between the first compressed data (Z2) and the second compressed data (Z2').

7. The detection method of the event of claim 6, **characterized in that** the physiological state is a heartbeat, the first compressed data (Z2) is obtained by encoding a sensing result of the heartbeat via the encoder (ECR), and the event is a sleep apnea event, wherein the sensing result of the heartbeat is an R-R interval (RRI) of a heartbeat waveform in a time sequence; or the motion state is an inertial attitude, the first compressed data (Z2) is obtained by encoding a sensing result of the inertial attitude via the encoder (ECR), and the event is a fall event.

8. The detection method of the event of any one of claims 6-7, **characterized in that** the step of determining the event comprises:

   determining whether the error is less than a threshold; wherein the threshold is obtained by a plurality of samples labeled as the event; the event occurs in response to the error being less than the threshold; and the event does not occur in response to the error not being less than the threshold.

9. The detection method of the event of any one of claims 6-8, **characterized in that** the step of receiving the first compressed data (Z2) comprises: receiving the first compressed data (Z2) via a low power wide area network, named LPWAN.

10. The detection method of the event of any one claims 6-9, **characterized in that** the detection method further comprises: training the anomaly detection model (ADM) by a plurality of samples labeled as the event.

11. An inference server (20), comprising:

   a communication transceiver (11) transmitting or receiving data;
   a memory (12) storing a program code; and
   a processor (14), **characterized in that** the processor (14) loads the program code to execute:

      receiving first compressed data (Z2) via the communication transceiver (11); wherein the first compressed data (Z2) is generated by encoding a sensing result of a physiological state or a motion state via an encoder (ECR);
      decoding the first compressed data (Z2) into reconstructed data (X2') via a decoder (DCR) in an anomaly detection model (ADM); wherein the anomaly detection model (ADM) is based on an autoencoder, and the anomaly detection model (ADM) comprises the decoder (DCR) and the encoder (ECR);
      encoding the reconstructed data (X2') into second compressed data (Z2') via the encoder (ECR); and
      determining an event of the physiological state or the motion state by an error between the first compressed data (Z2) and the second compressed data (Z2').

12. The inference server (20) of claim 11, **characterized in that** the physiological state is a heartbeat, a sensing result of the heartbeat is an R-R interval (RRI) of a heartbeat waveform in a time sequence, the first compressed data (Z2) is obtained by encoding the sensing result of the heartbeat via the encoder (ECR), and the event is a sleep apnea event; or the motion state is an inertial attitude, the first compressed data (Z2) is obtained by encoding a sensing result of the inertial attitude via the encoder (ECR), and the event is a fall event.

13. The inference server (20) of any one of claims 11-12, **characterized in that** the processor (14) further executes:

determining whether the error is less than a threshold; wherein the threshold is obtained by a plurality of samples labeled as the event; the event occurs in response to the error being less than the threshold; and the event does not occur in response to the error not being less than the threshold.

14. The inference server (20) of any one of claims 11-13, **characterized in that** the processor (14) further executes:
receiving the first compressed data (Z2) via a low power wide area network, named LPWAN, via the communication transceiver (11).

15. The inference server (20) of any one of claims 11-14, **characterized in that** the anomaly detection model (ADM) is trained by a plurality of samples labeled as the event; wherein the anomaly detection model (ADM) is trained based on an autoencoder.

**Patentansprüche**

1. Ereignisdetektionssystem (1), wobei das Ereignisdetektionssystem (1) umfasst:

ein Endgerät (10), das ein Erfassungsergebnis eines physiologischen Zustands oder eines Bewegungszustands über einen Kodierer (ECR) in erste komprimierte Daten (Z2) codiert; und einen Inferenzserver (20),

Empfangen der ersten komprimierten Daten (Z2) über ein Netzwerk; Dekodieren der ersten komprimierten Daten (Z2) über einen Dekoder (DCR) in einem Anomaliedetektionsmodell (ADM) in rekonstruierte Daten (X2'); wobei das Anomaliedetektionsmodell (ADM) den Dekoder (DCR) und den Kodierer (ECR) umfasst; Kodieren der rekonstruierten Daten (X2') über den Kodierer (ECR) in zweite komprimierte Daten (Z2'); und Bestimmen eines Ereignisses des physiologischen Zustands oder des Bewegungszustands anhand eines Fehlers zwischen den ersten komprimierten Daten (Z2) und den zweiten komprimierten Daten (Z2').

2. Ereignisdetektionssystem (1) gemäß Anspruch 1, **dadurch gekennzeichnet, dass** der physiologische Zustand ein Herzschlag ist und das Endgerät (10) ferner:

ein Erfassungsergebnis des Herzschlags über den Kodierer (ECR) in die ersten komprimierten Daten (Z2) codiert; wobei das Ereignis ein Schlafapnoe-Ereignis ist, wobei das Erfassungsergebnis des Herzschlags ein R-R-Intervall (RRI) einer Herzschlagwellenform in einer Zeitsequenz ist; oder
der Bewegungszustand eine Trägheitshaltung ist und das Endgerät (10) ferner:
ein Erfassungsergebnis der Trägheitshaltung über den Kodierer (ECR) in die ersten komprimierten Daten (Z2) codiert; wobei das Ereignis ein Sturzereignis ist.

3. Ereignisdetektionssystem (1) gemäß einem der Ansprüche 1 bis 2, **dadurch gekennzeichnet, dass** der Inferenzserver (20) ferner:

bestimmt, ob der Fehler kleiner als ein Schwellenwert ist; wobei der Schwellenwert durch eine Vielzahl von als Ereignis gekennzeichneten Abtastwerten erhalten wird; das Ereignis auftritt, als Reaktion darauf, dass der Fehler kleiner als der Schwellenwert ist; und das Ereignis nicht auftritt, als Reaktion darauf, dass der Fehler nicht kleiner als der Schwellenwert ist.

4. Ereignisdetektionssystem (1) gemäß einem der Ansprüche 1 bis 3, **dadurch gekennzeichnet, dass** der Inferenzserver (20) ferner:
die ersten komprimierten Daten (Z2) über ein Low Power Wide Area Network, LPWAN genannt, von dem Endgerät (10) empfängt.

5. Ereignisdetektionssystem (1) gemäß einem der Ansprüche 1 bis 4, **dadurch gekennzeichnet, dass** das Ereignisdetektionssystem (1) ferner umfasst:
ein Trainingsgerät (30), das das Anomaliedetektionsmodell (ADM) anhand einer Vielzahl von als das Ereignis gekennzeichneten Abtastwerten trainiert; wobei das Anomaliedetektionsmodell (ADM) basierend auf einen Autoencoder trainiert wird.

6. Ereignis-Detektionsverfahren, wobei das Detektionsverfahren umfasst:

Codieren eines Erfassungsergebnisses eines physiologischen Zustands oder eines Bewegungszustands in erste komprimierte Daten (Z2) über einen Kodierer (ECR);

Empfangen der ersten komprimierten Daten (Z2) über ein Netzwerk;

Decodieren der ersten komprimierten Daten (Z2) in rekonstruierte Daten (X2') über einen Dekoder (DCR) in einem Anomaliedetektionsmodell (ADM); wobei das Anomaliedetektionsmodell (ADM) auf einen Autoencoder basiert und das Anomaliedetektionsmodell (ADM) den Dekoder (DCR) und den Kodierer (ECR) umfasst;

Codieren der rekonstruierten Daten (X2') in zweite komprimierte Daten (Z2') über den Kodierer (ECR); und

Bestimmen eines Ereignisses des physiologischen Zustands oder des Bewegungszustands anhand eines Fehlers zwischen den ersten komprimierten Daten (Z2) und den zweiten komprimierten Daten (Z2').

7. Ereignis-Detektionsverfahren gemäß Anspruch 6, **dadurch gekennzeichnet, dass** der physiologische Zustand ein Herzschlag ist, die ersten komprimierten Daten (Z2) durch Codieren eines Erfassungsergebnisses des Herzschlags über den Kodierer (ECR) erhalten werden und das Ereignis ein Schlafapnoe-Ereignis ist, wobei das Erfassungsergebnis des Herzschlags ein R-R-Intervall (RRI) einer Herzschlagwellenform in einer Zeitsequenz ist; oder der Bewegungszustand eine Trägheitshaltung ist, die ersten komprimierten Daten (Z2) durch Codieren eines Erfassungsergebnisses der Trägheitshaltung über den Kodierer (ECR) erhalten werden und das Ereignis ein Sturzereignis ist.

8. Ereignis-Detektionsverfahren gemäß einem der Ansprüche 6 bis 7, **dadurch**

   **gekennzeichnet, dass** der Schritt des Bestimmens des Ereignisses umfasst:
   Bestimmen, ob der Fehler kleiner als ein Schwellenwert ist; wobei der Schwellenwert durch eine Vielzahl von als Ereignis gekennzeichneten Abtastwerten erhalten wird;
   das Ereignis auftritt, in Reaktion darauf, dass der Fehler kleiner als der Schwellenwert ist; und
   das Ereignis nicht auftritt, in Reaktion darauf, dass der Fehler nicht kleiner als der Schwellenwert ist.

9. Ereignis-Detektionsverfahren gemäß einem der Ansprüche 6 bis 8, **dadurch gekennzeichnet, dass** der Schritt des Empfangens der ersten komprimierten Daten (Z2) umfasst:
   Empfangen der ersten komprimierten Daten (Z2) über ein Low Power Wide Area Network, genannt LPWAN.

10. Ereignis-Detektionsverfahren gemäß einem der Ansprüche 6 bis 9, **dadurch gekennzeichnet, dass** das Detektionsverfahren ferner umfasst:
    Trainieren des Anomaliedetektionsmodells (ADM) anhand einer Vielzahl von als Ereignis gekennzeichneten Abtastwerten.

11. Inferenzserver (20), der umfasst:

    einen Kommunikations-Transceiver (11), der Daten sendet oder empfängt;
    einen Speicher (12), der einen Programmcode speichert; und
    einen Prozessor (14), **dadurch gekennzeichnet, dass** der Prozessor (14) den Programmcode lädt, um auszuführen:

    Empfangen erster komprimierter Daten (Z2) über den Kommunikations-Transceiver (11); wobei die ersten komprimierten Daten (Z2) durch Codieren eines Erfassungsergebnisses eines physiologischen Zustands oder eines Bewegungszustands über einen Kodierer (ECR) erzeugt werden;
    Decodieren der ersten komprimierten Daten (Z2) in rekonstruierte Daten (X2') über einen Dekoder (DCR) in einem Anomaliedetektionsmodell (ADM); wobei das Anomaliedetektionsmodell (ADM) auf einen Autoencoder basiert und das Anomaliedetektionsmodell (ADM) den Dekoder (DCR) und den Kodierer (ECR) umfasst;
    Codieren der rekonstruierten Daten (X2') in zweite komprimierte Daten (Z2') über den Kodierer (ECR); und
    Bestimmen eines Ereignisses des physiologischen Zustands oder des Bewegungszustands anhand eines Fehlers zwischen den ersten komprimierten Daten (Z2) und den zweiten komprimierten Daten (Z2').

12. Inferenzserver (20) gemäß Anspruch 11, **dadurch gekennzeichnet, dass** der physiologische Zustand ein Herzschlag ist, ein Erfassungsergebnis des Herzschlags ein R-R-Intervall (RRI) einer Herzschlagwellenform in einer Zeitsequenz ist, die ersten komprimierten Daten (Z2) durch Codieren des Erfassungsergebnisses des Herzschlags über den Kodierer (ECR) erhalten werden und das Ereignis ein Schlafapnoe-Ereignis ist; oder der Bewegungszustand eine Trägheitshaltung ist, die ersten komprimierten Daten (Z2) durch Codieren eines Erfassungsergebnisses der Trägheitshaltung über den Kodierer (ECR) erhalten werden und das Ereignis ein Sturzereignis ist.

13. Inferenzserver (20) gemäß einem der Ansprüche 11 bis 12, **dadurch gekennzeichnet, dass** der Prozessor (14) ferner ausführt:

Bestimmen, ob der Fehler kleiner als ein Schwellenwert ist; wobei der Schwellenwert durch eine Vielzahl von als Ereignis gekennzeichneten Abtastwerten erhalten wird; das Ereignis auftritt, in Reaktion darauf, dass der Fehler kleiner als der Schwellenwert ist; und das Ereignis nicht auftritt, in Reaktion darauf, dass der Fehler nicht kleiner als der Schwellenwert ist.

**14.** Inferenzserver (20) gemäß einem der Ansprüche 11 bis 13, **dadurch gekennzeichnet, dass** der Prozessor (14) ferner ausführt:
Empfangen der ersten komprimierten Daten (Z2) über ein Low Power Wide Area Network, genannt LPWAN, über den Kommunikationstransceiver (11).

**15.** Inferenzserver (20) gemäß einem der Ansprüche 11 bis 14, **dadurch gekennzeichnet, dass** das Anomaliedetektionsmodell (ADM) durch eine Vielzahl von als Ereignis gekennzeichneten Abtastwerten trainiert wird; wobei das Anomaliedetektionsmodell (ADM) basierend auf einen Autoencoder trainiert wird.

**Revendications**

**1.** Système de détection d'événement (1), dans lequel le système de détection d'événement (1) comprend :

un dispositif terminal (10) encodant un résultat de détection d'un état physiologique ou d'un état de mouvement en premières données compressées (Z2) via un encodeur (ECR) ; et
un serveur d'inférence (20) :

recevant les premières données compressées (Z2) via un réseau ;
décodant les premières données compressées (Z2) en données reconstruites (X2') via un décodeur (DCR) dans un modèle de détection d'anomalie (ADM) ; dans lequel le modèle de détection d'anomalie (ADM) comprend le décodeur (DCR) et l'encodeur (ECR) ;
encodant les données reconstruites (X2') en deuxièmes données compressées (Z2') via l'encodeur (ECR) ; et
déterminant un événement de l'état physiologique ou de l'état de mouvement par une erreur entre les premières données compressées (Z2) et les deuxièmes données compressées (Z2').

**2.** Système de détection d'événement (1) selon la revendication 1, **caractérisé en ce que** l'état physiologique est un rythme cardiaque, et le dispositif terminal (10) en outre :

encode un résultat de détection du rythme cardiaque en les premières données compressées (Z2) via l'encodeur (ECR) ; dans lequel l'événement est un événement d'apnée du sommeil, dans lequel le résultat de détection du rythme cardiaque est un intervalle R-R (RRI) d'une forme d'onde de rythme cardiaque dans une séquence chronologique ; ou
l'état de mouvement est une attitude inertielle, et le dispositif terminal (10) en outre :
encode un résultat de détection de l'attitude inertielle en les premières données compressées (Z2) via l'encodeur (ECR) ; dans lequel l'événement est un événement de chute.

**3.** Système de détection d'événement (1) selon l'une quelconque des revendications 1 et 2, **caractérisé en ce que** le serveur d'inférence (20) en outre :

détermine si l'erreur est ou non inférieure à un seuil ; dans lequel le seuil est obtenu par une pluralité d'échantillons marqués en tant que l'événement ;
l'événement survient en réponse au fait que l'erreur est inférieure au seuil ; et
l'événement ne survient pas en réponse au fait que l'erreur n'est pas inférieure au seuil.

**4.** Système de détection d'événement (1) selon l'une quelconque des revendications 1 à 3, **caractérisé en ce que** le serveur d'inférence (20) en outre :
reçoit les premières données compressées (Z2) depuis le dispositif terminal (10) via un réseau étendu à faible consommation d'énergie, appelé LPWAN.

**5.** Système de détection d'événement (1) selon l'une quelconque des revendications 1 à 4, **caractérisé en ce que** le système de détection d'événement (1) comprend en outre :
un dispositif d'entraînement (30) entraînant le modèle de détection d'anomalie (ADM) par une pluralité d'échantillons marqués en tant que l'événement ; dans lequel le modèle de détection d'anomalie (ADM) est entraîné sur la base d'un auto-encodeur.

**6.** Procédé de détection d'un événement, dans lequel le procédé de détection comprend :

l'encodage d'un résultat de détection d'un état physiologique ou d'un état de mouvement en premières données compressées (Z2) via un encodeur (ECR) ;
la réception des premières données compressées (Z2) via un réseau ;

le décodage des premières données compressées (Z2) en données reconstruites (X2') via un décodeur (DCR) dans un modèle de détection d'anomalie (ADM) ; dans lequel le modèle de détection d'anomalie (ADM) est basé sur un auto-encodeur, et le modèle de détection d'anomalie (ADM) comprend le décodeur (DCR) et l'encodeur (ECR) ;

l'encodage des données reconstruites (X2') en deuxièmes données compressées (Z2') via l'encodeur (ECR) ; et

la détermination d'un événement de l'état physiologique ou de l'état de mouvement par une erreur entre les premières données compressées (Z2) et les deuxièmes données compressées (Z2').

7. Procédé de détection de l'événement selon la revendication 6, **caractérisé en ce que** l'état physiologique est un rythme cardiaque, les premières données compressées (Z2) sont obtenues par l'encodage d'un résultat de détection du rythme cardiaque via l'encodeur (ECR), et l'événement est un événement d'apnée du sommeil, dans lequel le résultat de détection du rythme cardiaque est un intervalle R-R (RRI) d'une forme d'onde de rythme cardiaque dans une séquence chronologique ; ou

l'état de mouvement est une attitude inertielle, les premières données compressées (Z2) sont obtenues par l'encodage d'un résultat de détection de l'attitude inertielle via l'encodeur (ECR), et l'événement est un événement de chute.

8. Procédé de détection de l'événement selon l'une quelconque des revendications 6 et 7, **caractérisé en ce que** l'étape de détermination de l'événement comprend :

la détermination si l'erreur est ou non inférieure à un seuil ; dans lequel le seuil est obtenu par une pluralité d'échantillons marqués en tant que l'événement ;

l'événement survient en réponse au fait que l'erreur est inférieure au seuil ; et

l'événement ne survient pas en réponse au fait que l'erreur n'est pas inférieure au seuil.

9. Procédé de détection de l'événement selon l'une quelconque des revendications 6 à 8, **caractérisé en ce que** l'étape de réception des premières données compressées (Z2) comprend :
la réception des premières données compressées (Z2) via un réseau étendu à faible consommation d'énergie, appelé LPWAN.

10. Procédé de détection de l'événement selon l'une quelconque des revendications 6 à 9, **caractérisé en ce que** le procédé de détection comprend en outre :
l'entraînement du modèle de détection d'anomalie (ADM) par une pluralité d'échantillons marqués en tant que l'événement.

11. Serveur d'inférence (20), comprenant :

un émetteur-récepteur de communication (11) émettant ou recevant des données ;

une mémoire (12) stockant un code de programme ; et

un processeur (14), **caractérisé en ce que** le processeur (14) charge le code de programme pour exécuter :

la réception de premières données compressées (Z2) via l'émetteur-récepteur de communication (11) ; dans lequel les premières données compressées (Z2) sont générées par l'encodage d'un résultat de détection d'un état physiologique ou d'un état de mouvement via un encodeur (ECR) ;

le décodage des premières données compressées (Z2) en données reconstruites (X2') via un décodeur (DCR) dans un modèle de détection d'anomalie (ADM) ; dans lequel le modèle de détection d'anomalie (ADM) est basé sur un auto-encodeur, et le modèle de détection d'anomalie (ADM) comprend le décodeur (DCR) et l'encodeur (ECR) ;

l'encodage des données reconstruites (X2') en deuxièmes données compressées (Z2') via l'encodeur (ECR) ; et

la détermination d'un événement de l'état physiologique ou de l'état de mouvement par une erreur entre les premières données compressées (Z2) et les deuxièmes données compressées (Z2').

12. Serveur d'inférence (20) selon la revendication 11, **caractérisé en ce que** l'état physiologique est un rythme cardiaque, un résultat de détection du rythme cardiaque est un intervalle R-R (RRI) d'une forme d'onde de rythme cardiaque dans une séquence chronologique, les premières données compressées (Z2) sont obtenues par l'encodage du résultat de détection du rythme cardiaque via l'encodeur (ECR), et l'événement est un événement d'apnée du sommeil ; ou

l'état de mouvement est une attitude inertielle, les premières données compressées (Z2) sont obtenues par l'encodage d'un résultat de détection de l'attitude inertielle via l'encodeur (ECR), et l'événement est un événement de chute.

13. Serveur d'inférence (20) selon l'une quelconque des revendications 11 et 12, **caractérisé en ce que** le processeur (14) exécute en outre :

la détermination si l'erreur est ou non inférieure à un seuil ; dans lequel le seuil est obtenu par une pluralité d'échantillons marqués en tant que l'événement ;
l'événement survient en réponse au fait que l'erreur est inférieure au seuil ; et
l'événement ne survient pas en réponse au fait que l'erreur n'est pas inférieure au seuil.

14. Serveur d'inférence (20) selon l'une quelconque des revendications 11 à 13, **caractérisé en ce que** le processeur (14) exécute en outre :
la réception des premières données compressées (Z2) via un réseau étendu à faible consommation d'énergie, appelé LPWAN, via l'émetteur-récepteur de communication (11) .

15. Serveur d'inférence (20) selon l'une quelconque des revendications 11 à 14, **caractérisé en ce que** le modèle de détection d'anomalie (ADM) est entraîné par une pluralité d'échantillons marqués en tant que l'événement ; dans lequel le modèle de détection d'anomalie (ADM) est entraîné sur la base d'un auto-encodeur.

FIG. 1

S210 — Receive first compressed data

S220 — Decode the first compressed data into reconstructed data via a decoder in an anomaly detection model

S230 — Encode the reconstructed data into second compressed data via an encoder

S240 — Determine an event of a physiological state or a motion state by an error between the first compressed data and the second compressed data

FIG. 2

FIG. 3

FIG. 4

FIG. 5

FIG. 6

S710

Determine
whether an error is less than a
threshold

No

Yes

S720 — An event occurs

An event does not occur — S730

FIG. 7

**EP 4 410 184 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2022303288 A1
- CN 110876621 A
- US 2021244339 A1